# DEMANDE DE BREVET EUROPEEN

(11) **EP 1 649 895 A2**
(43) Date de publication de la demande: **26.04.2006**
(21) Numéro de dépôt: 05292160.8
(22) Date de dépôt: 13.10.2005
(51) Int. Cl.: A61Q 5/04, A61K 8/40, A61Q 5/08

(54) **Composition oxydante comprenant un monomere électrophile et un agent oxydant autre que la benzoquinone**

(30) Priorité: 13.10.2004 FR 0410804
(71) Demandeur: L'OREAL, 75008 Paris (FR)
(72) Inventeur: Plos, Grégory, c/o Gluck Heim Yoga 206, 158-0097 Tokyo (JP)
(74) Mandataire: Fevrier, Murielle Françoise E.

(57) **Abrégé**

La présente invention a pour objet une composition oxydante, en particulier pour la décoloration ou la déformation permanente des fibres kératiniques comprenant au moins un monomère électrophile et au moins un agent oxydant autre que la benzoquinone, un procédé de décoloration ou de déformation permanente des fibres kératiniques mettant en oeuvre une composition oxydante comprenant au moins un monomère électrophile et au moins un agent oxydant, ainsi que l'utilisation pour la décoloration ou la déformation permanente des fibres kératiniques d'une telle composition.

La présente invention permet d'obtenir un effet d'éclaircissement ou une déformation permanente des fibres kératiniques sans les dégrader et sans altérer leurs propriétés cosmétiques.

## Description

La présente invention a pour objet une composition oxydante, en particulier pour la décoloration ou la déformation permanente des fibres kératiniques tels que les cheveux, comprenant, dans un milieu cosmétiquement acceptable, au moins un monomère électrophile et au moins un agent oxydant autre que la benzoquinone.

Il est connu de décolorer les fibres kératiniques, et en particulier les fibres kératiniques humaines telles que les cheveux, avec des compositions de décoloration contenant un ou plusieurs agents oxydants et un ou plusieurs composés alcalins tels que les amines ou les silicates alcalins.

Les procédés de déformation permanente des fibres kératiniques connus de l'art antérieur consistent généralement en deux étapes. Dans la première étape, les fibres kératiniques sont mises en contact avec une composition réductrice afin de réduire les ponts di-sulfures présents dans ces fibres. Avant, après, ou simultanément à la réduction de ces ponts di-sulfures, le cheveu est mis en forme de la manière souhaitée. La deuxième étape est une étape de fixation nécessaire pour rétablir les ponts di-sulfures et ainsi stabiliser la forme donnée. Cette opération est habituellement effectuée à l'aide de compositions contenant un ou plusieurs agents oxydants.

Parmi les agents oxydants classiquement utilisés pour la coloration ou l'étape de fixation dans un procédé de déformation permanente des fibres kératiniques, on peut citer le peroxyde d'hydrogène ou des composés susceptibles de produire le peroxyde d'hydrogène par hydrolyse, tels que le peroxyde d'urée, ainsi que les persels comme les perborates, les percarbonates et les persulfates, le peroxyde d'hydrogène et les persulfates étant particulièrement préférés.

L'utilisation de tels agents oxydants présentent l'inconvénient de dégrader les fibres kératiniques et d'altérer leurs propriétés cosmétiques pour conduire par exemple à un démêlage difficile, un toucher désagréable ou des cheveux rêches et ternes.

Par ailleurs, il est connu de la demande de brevet FR 2 833 489 une composition pour le traitement des fibres kératiniques comprenant des monomères électrophiles. Une telle composition permet d'obtenir des cheveux parfaitement gainés et non gras.

Le but de la présente invention est de fournir des compositions oxydantes qui ne présentent pas les inconvénients des produits connus de l'art antérieur, en particulier des compositions oxydantes pour la décoloration des fibres kératiniques qui permettent d'obtenir un effet d'éclaircissement des fibres kératiniques sans dégrader les fibres kératiniques et sans altérer leurs propriétés cosmétiques, et des compositions oxydantes pour la déformation permanente des fibres kératiniques qui permettent d'obtenir une déformation permanente des fibres kératiniques sans dégrader les fibres kératiniques ni altérer leurs propriétés cosmétiques.

Ce but est atteint avec la présente invention qui a pour objet une composition oxydante comprenant, dans un milieu cosmétiquement acceptable, au moins un monomère électrophile et au moins un agent oxydant autre que la benzoquinone.

Lorsque la composition conforme à la présente invention est utilisée pour la décoloration des fibres kératiniques, elle permet d'obtenir un effet d'éclaircissement de ces fibres sans les dégrader et sans altérer leurs propriétés cosmétiques.

Lorsque la composition conforme à la présente invention est utilisée pour la déformation permanente des fibres kératiniques, elle permet d'obtenir une déformation permanente de ces fibres sans les dégrader et sans altérer leurs propriétés cosmétiques.

La présente invention a également pour objet un procédé de décoloration ou de déformation permanente des fibres kératiniques mettant en oeuvre une composition oxydante comprenant, dans un milieu cosmétiquement acceptable, au moins un monomère électrophile et au moins un agent oxydant, ainsi que des dispositifs à plusieurs compartiments pour la mise en oeuvre de ce procédé.

La présente invention a aussi pour objet l'utilisation pour la décoloration et la déformation permanente des fibres kératiniques d'une composition oxydante comprenant, dans un milieu cosmétiquement acceptable, au moins un monomère électrophile et au moins un agent oxydant.

Selon un mode de réalisation particulier de l'invention, le ou les agents oxydants sont choisis parmi :
- le peroxyde d'hydrogène ou un agent susceptible de produire du peroxyde d'hydrogène par hydrolyse tel que le peroxyde d'urée ;
- les persulfates, les perborates, les percarbonates d'ammonium ou de métaux alcalins ;
- les bromates alcalins ;
- le monoperoxyphthalate de magnésium ;
- les nitrites et plus particulièrement les nitrites de métaux alcalins, acalinoterreux ou d'ammonium, les dérivés organiques des nitrites. Les nitrites préférés sont le nitrite de sodium ou le nitrite d'ammonium ;
- l'acide périodique et ses sels hydrosolubles. Les sels préférés sont les sels de lithium, de sodium, de potassium, de rhubidium, de césium, de magnésium, de calcium, de strontium, de manganèse, de fer, de cuivre, de zinc, et d'aluminium. Sont plus particulièrement préférés les sels de sodium et de potassium ;
- les ferricyanures de métaux alcalins et en particulier le ferricyanure de potassium ;
- l'oxyde d'argent ;
- le réactif de Fenton;
- l'oxyde de plomb (IV);
- les anions d'un métal choisi parmi les permanganates ou les bichromates. Plus particulièrement sont préférés le permanganate de potassium et le dichromate de sodium ;
- les sels métalliques du groupe III à VIII du tableau périodique, tels que les sels de manganèse, de cobalt, de fer, de cuivre, d'argent, comme par exemple le sulfate de manganèse, le lactate de manganèse, le chlorure de cobalt, le chlorure ferrique, le chlorure cuivrique, le nitrate d'argent ammoniacal, les sels de cuivres qui sont préférés. Ces sels métalliques peuvent être à l'état libre ou adsorbés ;
- les sels de terres rares. Ceux-ci sont choisis parmi les sels de lanthanides, les sels de cérium Ce³⁺, Ce⁴⁺, les sels de lanthane La³⁺, d'europium Eu²⁺, Eu³⁺, les sels de gadolinium Gd³⁺, les sels d'Ytterbium Yb²⁺, Yb³⁺, de dysprosium Dy³⁺. Les sels préférés sont les sulfates, les chlorures ou les nitrates. De préférence, on choisira le Ce³⁺ ou le Ce⁴⁺ sous forme de sulfate ou de chlorure ;
- l'hypochlorite de sodium.

Selon un mode de réalisation préféré de l'invention, le ou les agents oxydants utiles dans le cadre de la présente invention sont choisis parmi le peroxyde d'hydrogène ou un agent susceptible de produire du peroxyde d'hydrogène par hydrolyse tel que le peroxyde d'urée.

Selon un autre mode de réalisation préféré de l'invention, le ou les agents oxydants sont choisis parmi les persulfates.

Le ou les agents oxydants sont généralement présents dans la composition conforme à l'invention en quantité comprise entre 0,05 % et 40 % en poids environ du poids total de la composition, de préférence entre 0,1 % et 35 % en poids environ du poids total de la composition, et encore plus préférentiellement entre 0,25 % et 25 % en poids environ du poids total de la composition.

Dans le cadre de la présente invention, le ou les monomères électrophiles sont choisis parmi les composés de formule (I) : 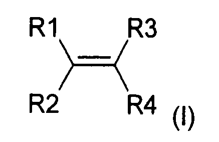 dans laquelle:
- R₁ et R₂ désignent chacun, indépendamment l'un de l'autre, un groupe peu ou non électro-attracteur (peu ou non inductif-attracteur) tel que:
   - un atome d'hydrogène,
   - un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR, -COOR, -COR, -SH, -SR, -OH, et les atomes d'halogène,
   - un résidu polyorganosiloxane modifié ou non,
   - un groupement polyoxyalkylène ;
- R₃ et R₄ désignent chacun, indépendamment l'un de l'autre, un groupe électro-attracteur (ou inductif-attacteur) choisi de préférence parmi les groupements -N(R)₃⁺, -S(R)₂⁺, -SH₂⁺, -NH₃⁺, -NO₂, -SO₂R, -C≡N, -COOH, -COOR, -COSR, -CONH₂, -CONHR, -F, -CI, -Br, -I, -OR, -COR, -SH, -SR, -OH, les groupes alcényle linéaires ou ramifiés, les groupes alcynyle linéaires ou ramifiés, les groupements mono- ou polyfluoroalkyle en C₁-C₄, les groupements aryle tels que phényle, ou les groupements aryloxy tels que phénoxyloxy ;
- R désigne un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', -COOR', -COR', -SH, -SR', -OH, les atomes d'halogène, ou un résidu de polymère pouvant être obtenu par polymérisation radicalaire, par condensation ou par ouverture de cycle, R' désignant un radical alkyle en C₁-C₁₀.

Par groupement électro-attracteur ou inductif-attracteur (-I), on entend tout groupement plus électronégatif que le carbone. On pourra se reporter à l'ouvrage PR Wells Prog. Phys. Org. Chem., Vol 6,111 (1968).

Par groupement peu ou non électro-attracteur, on entend tout groupement dont l'électronégativité est inférieure ou égale à celle du carbone.

Les groupements alcényle ou alcynyle ont de préférence 2 à 20 atomes de carbone, mieux encore de 2 à 10 atomes de carbone.

Comme groupe hydrocarboné saturé ou non, linéaire, ramifié ou cyclique, comportant de préférence de 1 à 20 atomes de carbone, mieux encore de 1 à 10 atomes de carbone, on peut notamment citer les groupes alkyle, alcényle ou alcynyle linéaires ou ramifiés, tels que méthyle, éthyle, n-butyle, tert-butyle, iso-butyle, pentyte, hexyle, octyle, butényle ou butynyle ; les groupes cycloalkyle ou aromatiques.

Comme groupe hydrocarboné substitué, on peut citer par exemple les groupes hydroxyalkyle ou polyhalogénoalkyle.

A titre d'exemples de polyorganosiloxane non modifié, on peut notamment citer les polyalkylsiloxanes tels que les polydiméthylsiloxanes, les polyarylsiloxanes tels que les polyphénylsiloxanes, les polyarylalkylsiloxanes tels que les polyméthylphénylsiloxanes.

Parmi les polyorganosiloxanes modifiés, on peut notamment citer les polydiméthylsiloxanes à groupements polyoxyalkylène et/ou siloxy et/ou silanol et/ou amine et/ou imine et/ou fluoroalkyle.

Parmi les groupements polyoxyalkylène, on peut notamment citer les groupements polyoxyéthylène et les groupements polyoxypropylène ayant de préférence 1 à 200 motifs oxyalkylénés.

Parmi les groupements mono- ou polyfluoroalkyle, on peut notamment citer des groupements tels que -(CH₂)ₙ-(CF₂)ₘ-CF₃ ou -(CH₂)ₙ-(CF₂)ₘ-CHF₂ avec n=1 à 20 et m= 1 à 20.

Les substituants R₁ à R₄ peuvent éventuellement être substitués par un groupement ayant une activité cosmétique. Les activités cosmétiques particulièrement utilisées sont obtenues à partir de groupements à fonctions colorantes, antioxydantes, filtres UV et conditionnantes.

A titre d'exemples de groupement à fonction colorante, on peut notamment citer les groupements azoiques, quinoniques, méthiniques, cyanométhiniques et triarylméthane.

A titre d'exemples de groupement à fonction antioxydante, on peut notamment citer les groupements de type butylhydroxyanisole (BHA), butylhydroxytoluène (BHT) ou vitamine E.

A titre d'exemples de groupement à fonction filtre UV, on peut notamment citer les groupements de types benzophénones, cinnamates, benzoates, benzylidène-camphres et dibenzoylméthanes.

A titre d'exemples de groupement à fonction conditionnante, on peut notamment citer les groupements cationiques et de type esters gras.

Le ou les monomères électrophiles présents dans la composition de l'invention peuvent par exemple être choisis parmi :
- le benzylidène malononitrile (A), le 2-(4-chloro-benzylidène)-malononitrile (A1), le 2-cyano-3-phényl acrylate d'éthyle (B), le 2-cyano-3-(4-chloro-phényl) acrylate d'éthyle (B1) décrits dans Sayyah, *J*. *Polymer Research,* 2000, p. 97 : 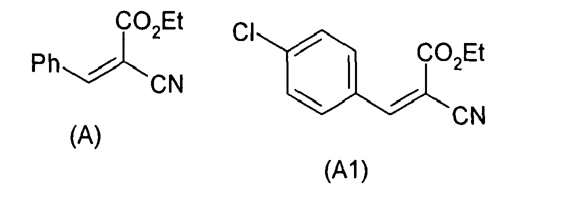 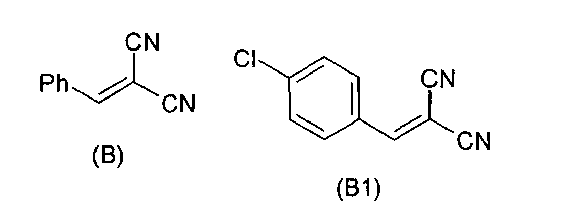
- les dérivés de méthylidène malonates comme le 2-méthylène-malonate de diéthyle (C) décrit dans Hopff, *Makromoteculare Chemie,* 1961, p. 95 ; De Keyser, *J*. *Pharm. Sci,* 1991, p. 67 et Klemarczyk, *Polymer,* 1998, p. 173 : 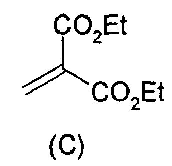
- le 2-éthoxycarbonylméthylèneoxycarbonyl acrylate d'éthyle (D) décrit dans Breton, *Biomaterials,* 1998, p. 271 et Couvreur, *Pharmaceutical Research,* 1994, p. 1270 : 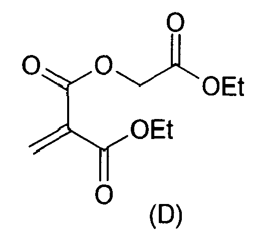
- les dérivés α-(méthylsulfonyl)acrylates de méthyle (K), α-(méthylsulfonyl)acrylates de d'éthyle (L), α-(tert-butylsulfonyl)acrylates de méthyle (M), α-(méthylsulfonyl)acrylates de tert-butyle (N), α-(tert-butylsulfonyl)acrylates de tert-butyle (O) décrits dans Gipstein, *J. Org. Chem,* 1980, p. 1486, et
- les dérivés 1,1-bis-(méthylsulfonyl)éthylène (P), 1-acétyl-1-méthyl sulfonyl éthylène (Q), α-(méthylsulfonyl) vinyl sulfonate de méthyle (R), α-méthylsulfonylacrylonitrile (S) décrits dans le brevet US 2 748 050 :

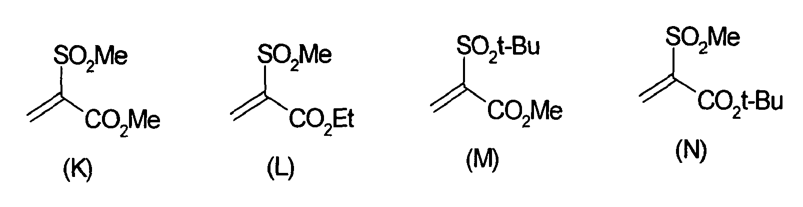

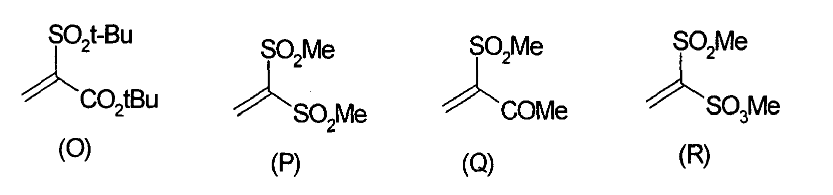

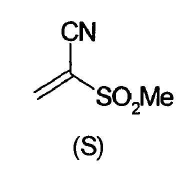

Selon un mode de réalisation préféré de l'invention, le ou les monomères électrophiles sont choisis parmi les monomères de la famille des cyanoacrylates de formule (II) : 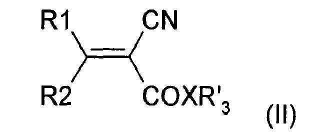 dans laquelle :
- X peut désigner NH, S, O ;
- R'₃ peut désigner un atome d'hydrogène ou un groupe R;
- R, R₁ et R₂ sont tels que définis précédemment.

Dans les formules (I) et (II), R₁ et R₂ représentent de préférence un atome d'hydrogène.

Dans la formule (II), X désigne de préférence O et R'₃ représente de préférence un radical alkyle en C₆-C₁₀.

A titre de composés de formule (II), on peut citer les monomères :
a) appartenant à la famille des 2-cyanoacrylates de polyfluoroalkyle tels que l'ester 2,2,3,3-tétrafluoropropylique de l'acide 2-cyano-2-propénoïque de formule (III): 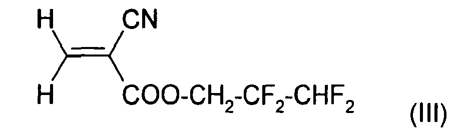 ou encore l'ester 2,2,2-trifluoroéthylique de l'acide 2-cyano-2-propénoïque de formule (IV) : 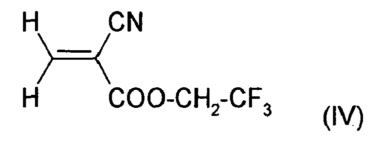
b) appartenant à la famille des 2-cyanoacrylates d'alkyle ou d'alcoxyalkyle de formule (V) : 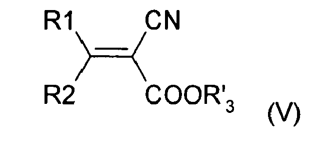 dans laquelle :
   - R'₃ représente un radical alkyle en C₁-C₁₀ ou alcoxy(C₁-C₄)alkyle(C₁₋C₁₀) ;
   - R₁ et R₂ sont tels que définis précédemment.

On peut citer plus particulièrement le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle, le 2-cyanoacrylate de n-octyle et le cyanoacrylate d'iso-amyle.

Dans la formule (V) telle que définie ci-dessus, R'₃ représente de préférence un radical alkyle en C₆-C₁₀, et R₁ et R₂ représentent un atome d'hydrogène.

Dans le cadre de l'invention, on préfère utiliser les monomères définis en b).

Les monomères le plus particulièrement préféré sont les composés de formule (VI) et leurs mélanges : 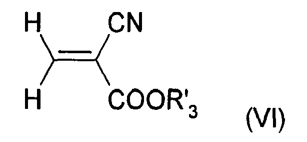 dans laquelle R'₃ est choisi parmi les radicaux suivants :
-(CH₂)₇-CH₃ ;
-CH(CH₃)-(CH₂)₅-CH₃ ;
-CH₂-CH(C₂H₅)-(CH₂)₃-CH₃ ;
-(CH₂)₅-CH(CH₃)-CH₃ ;
-(CH₂)₄-CH(C₂H₅)-CH₃.

Les monomères utilisés conformément à l'invention peuvent être fixés de façon covalente sur des supports tels que des polymères, des oligomères ou des dendrimères. Le polymère ou l'oligomère peut être linéaire, ramifié, en peigne ou bloc. La répartition des monomères de l'invention sur la structure polymérique, oligomérique ou dendritique peut être statistique, en position terminale ou sous forme de blocs.

Le ou les monomères électrophiles sont généralement présents dans la composition conforme à l'invention en quantité comprise entre 0,1 % et 80 % en poids environ du poids total de la composition, et de préférence entre 1 % et 50 % en poids environ du poids total de la composition.

Le ou les monomères électrophiles utiles dans le cadre de la présente invention peuvent être synthétisés selon les méthodes connues décrites dans la littérature. En particulier, les monomères de la famille des cyanoacrylates peuvent être synthétisés selon l'enseignement des documents US 3 527 224, US 3 591 767, US 3 667 472, US 3 995 641, US 4 035 334 et US 4 650 826.

Le ou les monomères électrophiles utiles dans le cadre de la présente invention sont des monomères capables de polymériser par polymérisation anionique en présence d'un agent nucléophile.

Par polymérisation anionique, on entend le mécanisme défini dans l'ouvrage « Advanced Organic Chemistry », Third Edition de Jerry March, pages 151 à 161.

Les agents nucléophiles susceptibles d'initier la polymérisation anionique sont des systèmes connus capables de générer un carbanion au contact d'un agent électrophile, tels que les ions hydroxyde contenus dans l'eau. On entend par carbanion les espèces chimiques définies dans l'ouvrage « Advanced Organic Chemistry », Third Edition de Jerry March, page 141.

Le ou les agents nucléophiles peuvent être appliqués indépendamment de la composition de l'invention. Ils peuvent aussi être ajoutés à la composition de l'invention au moment de l'emploi. Dans ce cas-là, la composition conforme à l'invention comprend de plus au moins un agent nucléophile.

Selon un mode de réalisation particulier de l'invention, le ou les agents nucléophiles sont choisis parmi las composés moléculaires, les oligomères, les dendrimères et les polymères portant au moins une fonction nucléophile choisie parmi les fonctions R₂N⁻, NH₂⁻, Ph₃C⁻, R₃C⁻, PhNH⁻, pyridine, ArS⁻, R-C≡C⁻, RS⁻, SH⁻, RO⁻, R₂NH, ArO⁻, N₃⁻, OH⁻, ArNH₂, NH₃, l⁻, Br⁻, Cl⁻, RCOO⁻, SCN⁻, ROH, RSH, NCO⁻, CN⁻, NO₃⁻, ClO₄⁻, H₂O, Ph représentant un groupe phényle, Ar représentant un groupe aryle et R représentant un groupe alkyle en C₁-C₁₀.

De préférence, l'agent nucléophile est l'eau.

Selon la présente invention, les monomères sont de préférence choisis parmi les monomères capables de polymériser sur les fibres kératiniques dans des conditions cosmétiquement acceptables. En particulier, la polymérisation du monomère s'effectue de préférence à une température inférieure ou égale à 80°C, de préférence entre 10 et 80°C, de préférence 20 à 80°C, ce qui n'empêche pas de terminer l'application par un séchage au casque, un brushing ou passage au fer plat ou à friser.

Le milieu cosmétiquement acceptable de la composition de l'invention est de préférence sous forme d'un milieu anhydre et non hygroscopique. On entend par milieu anhydre un milieu contenant moins de 1 % d'eau.

Le milieu cosmétiquement acceptable de la composition de l'invention est de préférence choisi parmi :
- les alcools aromatiques tels que l'alcool benzylique ;
- les alcools gras ;
- les polyols modifiés ou non tels que le glycérol, le glycol, le propylène glycol, le dipropylène glycol, le butylène glycol, le butyle diglycol ;
- les silicones volatiles ou non telles que la cyclopentasiloxane, la cyclohexasiloxane, les polydiméthylsiloxanes modifiées ou non par des fonctions phényle et / ou siloxy et / ou silanol et / ou amine et / ou imine et / ou fluoroalkyle et / ou carboxylique et / ou bétaïne et / ou ammonium quaternaire ;
- les huiles minérales, organiques ou végétales ;
- les cires oxyéthylénées ou non, les paraffines, les alcanes et plus particulièrement les alcanes en C₅-C₁₀;
- les acides gras, les amides grasses, les esters gras et plus particulièrement les benzoates ou les salicylates d'alcool gras ; et leurs mélanges.

Le milieu cosmétiquement acceptable de la composition de l'invention peut aussi se présenter sous forme d'une émulsion directe ou inverse et / ou être encapsulé, les monomères électrophiles étant maintenus dans un milieu anhydre jusqu'au moment de l'emploi. La phase dispersée ou continue de l'émulsion peut être constituée par de l'eau, un alcool aliphatique en C₁-C₄, des silicones ou leurs mélanges. Les capsules ou microcapsules contenant la composition de l'invention peuvent être dispersées dans un milieu anhydre tel que défini précédemment, de l'eau, un alcool aliphatique en C₁-C₄ ou leurs mélanges.

La composition conforme à l'invention peut de plus comprendre au moins un inhibiteur de polymérisation.

De préférence, le ou les inhibiteurs de polymérisation sont des inhibiteurs de polymérisation anionique et/ou radicalaire.

A titre d'exemples d'inhibiteurs de polymérisation anionique et /ou radicalaire, on peut citer le dioxyde de soufre, l'oxyde nitrique, les acides organiques tels qu'un acide sulfonique, l'acide phosphorique ou l'acide acétique, la lactone, le trifluorure de bore, l'hydroquinone et ses dérivés tels que l'hydroquinone monoéthyléther, la tertiobutyl hydroquinone, la benzoquinone et ses dérivés tels que la duroquinone, le catéchol et ses dérivés tels que le t-butyl catéchol et le méthoxycatéchol, l'anisole et ses dérivés tels que le méthoxyanisole ou l'hydroxyanisole, le pyrogallol et ses dérivés, le p-méthoxyphénol, l'hydroxybutyl toluène, les alkyl sulfates, les alkyl sulfites, les alkyl sulfones, les alkyl sulfoxydes, les alkyl sulfures, les mercaptans, le 3-sulfonène et leurs mélanges. Les groupements alkyle désignent de préférence des groupements ayant de 1 à 6 atomes de carbone.

Le ou les inhibiteurs de polymérisation sont généralement présents dans la composition conforme à l'invention en quantité comprise entre 0,01 % et 10 % en poids environ du poids total de la composition, et encore plus préférentiellement entre 0,05 % et 5 % en poids environ du poids total de la composition.

La composition conforme à l'invention peut de plus comprendre au moins un polymère épaississant ne présentant pas de réactivité sur le ou les monomères électrophiles utiles dans le cadre de la présente invention.

A titre d'exemples de polymères épaississants ne présentant pas de réactivité sur le ou les monomères électrophiles utilisés dans le cadre de l'invention, on peut citer de façon non exhaustive le polyméthylméthacrylate (PMMA) ou encore les copolymères à base de cyanoacrylate tels qu'ils sont décrits dans le brevet US 6 224 622.

Le ou les polymères épaississants sont généralement présents dans la composition conforme à l'invention en quantité comprise entre 0,1 % et 50 %, de préférence entre 0,5 % et 25 %. Ils sont entre autres utiles pour moduler la vitesse de polymérisation des monomères électrophiles.

La composition conforme à l'invention peut également contenir des composés additionnels utilisés classiquement en cosmétique. Ces composés peuvent être choisis parmi les agents réducteurs, les corps gras, les silicones organomodifiées ou non, les agents épaississants autres que les polymères épaississants définis ci-dessus, les polymères cationiques, anioniques, neutres ou amphotères, les adoucissants, les agents anti-mousse, les agents hydratants, les agents émollients, les agents alcalinisants, les agents anti-oxydants, les agents anti-radicaux libres, les agents chélatants, les agents antipelliculaires, les agents régulateurs de séborrhée, les agents apaisants, les plastifiants, les filtres solaires, les colorants directs (naturels ou non) ou d'oxydation (bases et /ou coupleurs), les pigments, les charges minérales, les argiles, les minéraux colloïdaux, les nacres, les parfums, les peptisants, les conservateurs, les tensioactifs anioniques, cationiques, amphotères, zwittérioniques ou non-ioniques, les polymères fixants ou non, les polymères conditionneurs, les protéines hydrolysées ou non, les enzymes, les acides aminés, les oligopeptides, les peptides, les vitamines, les saccharides, les oligosaccharides, les polysaccharides hydrolysés ou non, modifiés ou non, les polyacides amines, les alcools gras ramifiés ou non, les cires animales, végétales ou minérales, les céramides, les pseudo-céramides, les acides organiques hydroxylés, les polyisobutènes et poly(α-oléfines), les esters gras, les polymères anioniques sous forme dissoute ou dispersée, les polymères non ioniques sous forme dissoute ou dispersée.

Bien entendu, l'homme de l'art veillera à choisir ce ou ces éventuels composés additionnels de manière telle que les propriétés avantageuses attachées intrinsèquement à la composition conforme à l'invention ne soient pas, ou substantiellement pas, altérer par la ou les adjonctions envisagées.

La composition conforme à l'invention peut se présenter sous des formes diverses, telles que des lotions, des sprays, des mousses et être appliquées sous forme de shampooing ou d'après-shampooing.

La composition conforme à l'invention peut également contenir un propulseur. Le propulseur est généralement constitué par un gaz comprimé ou liquéfié usuellement employé pour la préparation de compositions aérosols. De préférence, le gaz comprimé ou liquéfié est choisi parmi l'air, le gaz carbonique, l'azote comprimé, un gaz soluble tel que le diméthyléther, les hydrocarbures halogénés tels que les hydrocarbures fluorés ou non halogénés, et leurs mélanges.

Le procédé selon la présente invention comprend l'application sur les fibres kératiniques d'une composition comprenant, dans un milieu cosmétiquement acceptable, au moins un monomère électrophile tel que défini précédemment et au moins un agent oxydant en présence d'au moins un agent nucléophile.

Le ou les agents oxydants peuvent être parmi :
- le peroxyde d'hydrogène ou un agent susceptible de produire du peroxyde d'hydrogène par hydrolyse tel que le peroxyde d'urée ;
- les persulfates, les perborates, les percarbonates d'ammonium ou de métaux alcalins ;
- les bromates alcalins ;
- le monoperoxyphthalate de magnésium ;
- les nitrites et plus particulièrement les nitrites de métaux alcalins, acalinoterreux ou d'ammonium, les dérivés organiques des nitrites. Les nitrites préférés sont le nitrite de sodium ou le nitrite d'ammonium ;
- l'acide périodique et ses sels hydrosolubles. Les sels préférés sont les sels de lithium, de sodium, de potassium, de rhubidium, de césium, de magnésium, de calcium, de strontium, de manganèse, de fer, de cuivre, de zinc, et d'aluminium. Sont plus particulièrement préférés les sels de sodium et de potassium ;
- les ferricyanures de métaux alcalins et en particulier le ferricyanure de potassium ;
- l'oxyde d'argent;
- le réactif de Fenton;
- l'oxyde de plomb (IV);
- les anions d'un métal choisi parmi les permanganates ou les bichromates. Plus particulièrement sont préférés le permanganate de potassium et le dichromate de sodium ;
- les sels métalliques du groupe III à VIII du tableau périodique, tels que les sels de manganèse, de cobalt, de fer, de cuivre, d'argent, comme par exemple le sulfate de manganèse, le lactate de manganèse, le chlorure de cobalt, le chlorure ferrique, le chlorure cuivrique, le nitrate d'argent ammoniacal, les sels de cuivres qui sont préférés. Ces sels métalliques peuvent être à l'état libre ou adsorbés ;
- les sels de terres rares. Ceux-ci sont choisis parmi les sels de lanthanides, les sels de cérium Ce³⁺, Ce⁴⁺, les sels de lanthane La³⁺, d'europium Eu²⁺, Eu³⁺, les sels de gadolinium Gd³⁺, les sels d'Ytterbium Yb²⁺, Yb³⁺, de dysprosium Dy³⁺. Les sels préférés sont les sulfates, les chlorures ou les nitrates. De préférence, on choisira le Ce³⁺ ou le Ce⁴⁺ sous forme de sulfate ou de chlorure ;
- l'hypochlorite de sodium ;
- la benzoquinone.

Selon une variante du procédé de l'invention, l'application de la composition conforme à l'invention est réalisée en au moins deux étapes, une des étapes consiste à appliquer sur les fibres kératiniques une composition comprenant le ou les monomères électrophiles et l'autre étape consiste à appliquer sur les fibres kératiniques une composition comprenant le ou les agents oxydants, l'ordre des étapes étant indifférent.

L'agent nucléophile utile peut être utilisé pur, en solution, sous forme d'une émulsion ou être encapsulé. Il peut être présent dans la composition contenant l'agent oxydant. Il peut aussi être appliqué séparément. Dans ce cas, il est possible par exemple d'imprégner préalablement les fibres kératiniques à l'aide de cet agent nucléophile.

Dans le cas où l'agent nucléophile est de l'eau, il est possible d'humidifier préalablement les fibres kératiniques à l'aide d'une solution aqueuse dont le pH a été ajusté à l'aide d'agents acidifiants et / ou alcalinisants.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (VII) suivante : 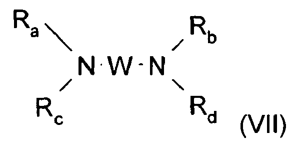 dans laquelle W est un reste propylène éventuellement substitué par un groupement hydroxyle ou un radical alkyle en C₁-C₄; Rₐ, R_{b}, R_{c} et R_{d}, identiques ou différents, représentent un atome d'hydrogène, un radical alkyle en C₁-C₄ ou hydroxyalkyle en C₁-C₄.

Selon un mode de réalisation particulier de l'invention, on augmente la nucléophilie des fibres kératiniques par transformation chimique de la matière kératinique. A titre d'exemple, on peut appliquer sur les fibres kératiniques au moins un agent réducteur de la kératine afin de réduire les ponts disulfure composant en partie la kératine en thiols. Le ou les agents réducteurs peuvent en particulier être appliqués sur les fibres kératiniques avant la composition comprenant le ou les agents oxydants.

Parmi les agents réducteurs de la kératine utiles, on peut citer:
- le thiosulfate de sodium anhydre;
- le métabisulfite de sodium en poudre;
- la thiourée;
- le sulfite d'ammonium;
- l'acide thioglycolique;
- l'acide thiolactique;
- le thiolactate d'ammonium;
- le mono-thioglycolate de glycérol ;
- le thioglycolate d'ammonium ;
- le thioglycérol;
- l'acide 2,5-dihydroxybenzoique;
- le di-thioglycolate de diammonium;
- le thioglycolate de strontium;
- le thioglycolate de calcium;
- le formo-sulfoxylate de zinc;
- le thioglycolate d'isooctyle;
- la dl-cystéine;
- le thioglycolate de monoéthanolamine.

Afin d'améliorer entre autres l'adhésion du polycyanoacrylate formé in situ, on peut préalablement traiter les fibres kératiniques avec tous types de polymères, ou réaliser un traitement capillaire, comme une coloration directe ou d'oxydation, une permanente ou un défrisage.

L'application des compositions sur les fibres kératiniques peut être suivie ou non d'un rinçage et / ou d'un séchage. Le séchage peut être effectué au casque, au sèche cheveux et / ou au fer à lisser.

Ces compositions peuvent en outre contenir divers composés additionnels tels que définis précédemment.

Selon le procédé conforme à l'invention, il est possible de réaliser des superpositions multiples d'applications.

La présente invention a aussi pour objet un kit pour la décoloration ou la déformation permanente des fibres kératiniques, caractérisé par le fait qu'il contient une composition comprenant au moins un monomère électrophile tel que défini précédemment et éventuellement au moins un inhibiteur de polymérisation et une composition comprenant au moins un agent oxydant tel que défini précédemment.

Le ou les monomères sont présents dans la composition les comprenant dans une concentration généralement comprise entre 0,05 et 30 % en poids environ du poids total de la composition, de préférence entre 0,01 et 50 % en poids, et encore plus préférentiellement entre 0,1 et 20 % en poids.

Selon un mode de réalisation particulier du kit conforme à l'invention, la composition comprenant le ou les agents oxydants comprend de plus au moins un agent nucléophile tel que défini précédemment.

Selon un autre mode de réalisation particulier du kit conforme à l'invention, le kit contient de plus une composition comprenant au moins un agent nucléophile tel que défini précédemment.

La présente invention a également pour objet l'utilisation pour la décoloration ou la déformation permanente des fibres kératiniques d'une composition telle que définie précédemment.

L'invention va être plus complètement illustrée à l'aide des exemples non limitatifs suivants.

### EXEMPLES

Les exemples 1 et 2 ci-dessous ont été réalisés en utilisant les composés suivants :
Monomère : n-octylcyanoacrylate stabilisé par 1 % d'acide phosphorique.
Milieu cosmétique :
   - 50 % d'un mélange poly diméthyl siloxane alpha-omega dihydroxylé /cyclopentadiméthylsiloxane (14,7 / 85,3) commercialisé par Dow Corning sous le nom de DC 1501 Fluid ;
   - 50 % de cyclopentadiméthylsiloxane commercialisé par Dow Corning sous le nom de DC245 Fluid.

### Exemple 1 :

1) On prépare une composition pour la décoloration des cheveux comprenant dans le milieu cosmétique 4,5 % en poids de persulfate de potassium, 50 % en poids d'eau oxygénée (6 % en poids), 3 % en poids d'ammoniaque (à 20,5 % en NH₃) et 10 % en poids de monomère.
2) 5 g de la composition ainsi obtenue sont appliqués sur une mèche de cheveu châtain naturels de 1 g, pendant 30 minutes et à température ambiante.
3) Apres 30 minutes de pose, la mèche est rincée à l'eau claire puis séchée au sèche-cheveux.

La mèche obtenue est décolorée et présente un toucher très cosmétique.

### Exemple 2 :

1) Une composition aqueuse est préparée avec 50 % en poids d'eau oxygénée 20 volumes et 10 % en poids d'ammoniaque (à 40 % en poids dans l'eau).
2) 5 g de cette composition aqueuse sont appliqués sur une mèche de cheveux châtains de 1 g, pendant 30 minutes et à température ambiante.
3) Après 30 minutes de pose, la mèche est rincée à l'eau claire.
4) 0,5 g d'une solution du milieu cosmétique contenant 10 % de monomère est appliquée sur la mèche.
5) Après 10 minutes de pose à température ambiante, la mèche est séchée pendant 2 minutes au sèche-cheveux.

La mèche obtenue est décolorée et présente un toucher très cosmétique.

### Exemples 3 à 8 :

Les compositions pour la décoloration des cheveux suivantes sont préparées (quantités en grammes) :

| Exemple | 3 | 4 | 5 | 6 | 7 | 8 |
|---|---|---|---|---|---|---|
| Méthylheptylcyanoacrylate commercialisé par la société Chemence | 10 | - | - | - | 9 | 7 |
| Ethoxyéthylcyanoacrylate EO 460 commercialisé par la société Tong Shen | - | 10 | - | - | - | - |
| Butylcyanoacrylate B 60 commercialisé par la société Tong Shen | - | - | 10 | - | - | 3 |
| Ethylhexylcyanoacrylate O 60 commercialisé par la société Tong Shen | - | - | - | 10 | 1 | - |
| DC 245 Fluid | 33,75 | 33,75 | 33,75 | 33,75 | 33,75 | 33,75 |
| DC 1501 Fluid | 33,75 | 33,75 | 33,75 | 33,75 | 33,75 | 33,75 |
| Persulfate de potassium | 4,5 | 4,5 | 4,5 | 4,5 | 4,5 | 4,5 |
| Eau oxygénée à 6 % en H₂O₂ | 50 | 50 | 50 | 50 | 50 | 50 |
| Ammoniaque à 20,5 % en NH₃ | 3 | 3 | 3 | 3 | 3 | 3 |

Chacune de ces compositions est appliquée sur une mèche de cheveux châtain naturels de 1 g, pendant 30 minutes et à température ambiante. La mèche est ensuite rincée puis séchée.

La mèche obtenue est décolorée et ses propriétés cosmétiques ne sont pas altérées.

### Exemple 9 :

Les compositions suivantes sont réalisées (quantités en grammes):
Composition A : composition contenant le monomère électrophile

| | |
|---|---|
| Méthylheptylcyanoacrylate commercialisé par la société Chemence | 10 |
| DC 245 Fluid | 45 |
| DC 1501 Fluid | 45 |

Composition B : composition décolorante

| | |
|---|---|
| Eau oxygénée à 6 % en H₂O₂ | 50 |
| Ammoniaque à 20,5 % en NH₃ | 10 |

La composition A est appliquée sur une mèche de cheveux châtain naturels de 1 g, propre et humide, pendant 15 minutes et à température ambiante, puis la mèche est séchée. La composition B est ensuite appliquée sur la mèche pendant 30 minutes à température ambiante, puis la mèche est rincée puis séchée.

La mèche obtenue est décolorée et ses propriétés cosmétiques ne sont pas altérées.

### Exemple 10 :

Les compositions suivantes sont réalisées (quantités en grammes) :
Composition C : composition contenant un réducteur

| | |
|---|---|
| Acide thioglycolique | 9 |
| Ammoniaque | q.s.p. pH 9 |
| Eau | q.s.p. 100 |

Composition D : composition contenant un oxydant

| | |
|---|---|
| Eau oxygénée à 50 % en H₂O₂ | 4,8 |
| Acide citrique | q.s.p. pH 3 |
| Eau | q.s.p. 100 |

Composition E : composition contenant le monomère électrophile

| | |
|---|---|
| Méthylheptylcyanoacrylate commercialisé par la société Chemence | 10 |
| DC 245 Fluid | 45 |
| DC 1501 Fluid | 45 |

La composition C est appliquée sur une mèche de cheveux châtain naturels de 1 g, propre et humide, enroulée sur un bigoudi, pendant 15 minutes et à température ambiante, puis la mèche est rincée. On applique ensuite la composition D sur la mèche pendant 15 minutes à température ambiante, puis la mèche est rincée. On applique ensuite la composition E sur la mèche pendant 15 minutes à température ambiante, puis la mèche est séchée et déroulée du bigoudi.

La mèche obtenue est mise en forme sans altération de ses propriétés cosmétiques.

### Exemple 11 :

Les compositions suivantes sont réalisées (quantités en grammes) :
Composition F : composition contenant le monomère électrophile

| | |
|---|---|
| Méthylheptylcyanoacrylate commercialisé par la société Chemence | 10 |
| DC 245 Fluid | 45 |
| DC 1501 Fluid | 45 |

Composition G : composition contenant un réducteur

| | |
|---|---|
| Acide thioglycolique | 9 |
| Ammoniaque | q.s.p. pH 9 |
| Eau | q.s.p. 100 |

Composition H : composition contenant un oxydant

| | |
|---|---|
| Eau oxygénée à 50 % en H₂O₂ | 4,8 |
| Acide citrique | q.sp. pH 3 |
| Eau | q.s.p. 100 |

La composition F est appliquée sur une mèche de cheveux châtain naturels de 1 g, propre et humide, pendant 15 minutes et à température ambiante, puis la mèche est séchée, humidifiée à nouveau puis enroulée sur un bigoudi. La composition G est alors appliquée sur la mèche pendant 15 minutes à température ambiante, puis la mèche est rincée. La composition H est ensuite appliquée sur la mèche pendant 15 minutes à température ambiante, puis la mèche est rincée et déroulée du bigoudi.

La mèche est mise en forme sans altération de ses propriétés cosmétiques.

## Revendications

1. Composition oxydante comprenant, dans un milieu cosmétiquement acceptable :
- au moins un monomère électrophile choisi parmi les composés de formule (I): 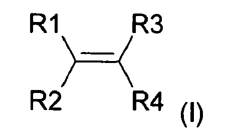
dans laquelle :
- R₁ et R₂ désignent chacun, indépendamment l'un de l'autre, un groupe peu ou non électro-attracteur ; et
- R₃ et R₄ désignent chacun, indépendamment l'un de l'autre, un groupe électro-attracteur ; et
- au moins un agent oxydant autre que la benzoquinone.

2. Composition selon la revendication 1, dans laquelle le ou les agents oxydants sont choisis parmi :
• le peroxyde d'hydrogène ou un agent susceptible de produire du peroxyde d'hydrogène par hydrolyse ;
• les persulfates, les perborates, les percarbonates d'ammonium ou de métaux alcalins ;
• les bromates alcalins ;
• le monoperoxyphthalate de magnésium ;
• les nitrites ;
• l'acide périodique et ses sels hydrosolubles ;
• les ferricyanures de métaux alcalins ;
• l'oxyde d'argent ;
• le réactif de Fenton ;
• l'oxyde de plomb (IV);
• les anions d'un métal choisi parmi les permanganates ou les bichromates ;
• les sels métalliques du groupe III à VIII du tableau périodique ;
• les sels de terres rares choisis parmi les sels de lanthanides, les sels de cérium Ce³⁺, Ce⁴⁺, les sels de lanthane La³⁺, d'europium Eu²⁺, Eu³⁺, les sels de gadolinium Gd³⁺, les sels d'Ytterbium Yb²⁺, Yb³⁺, de dysprosium Dy³⁺ ;
• l'hypochlorite de sodium.

3. Composition selon la revendication 2, dans laquelle le ou les agents oxydants sont choisis parmi le peroxyde d'hydrogène et les agents susceptibles de produire du peroxyde d'hydrogène par hydrolyse.

4. Composition selon la revendication 3, dans laquelle l'agent susceptible de produire du peroxyde d'hydrogène par hydrolyse est le peroxyde d'urée.

5. Composition selon la revendication 2, dans laquelle le ou les agents oxydants sont choisis parmi les persulfates.

6. Composition selon l'une quelconque des revendications précédentes, dans laquelle le ou les agents oxydants sont présents en quantité comprise entre 0,05 % et 40 % en poids environ du poids total de la composition.

7. Composition selon l'une quelconque des revendications précédentes, dans laquelle R₁ et R₂ représentent, indépendamment l'un de l'autre, un atome d'hydrogène ; un groupe hydrocarboné saturé ou non, linéaire, ramifié ou cyclique, comportant de 1 à 20 atomes de carbone et contenant éventuellement un ou plusieurs atomes d'azote, d'oxygène, de soufre, et éventuellement substitué par un ou plusieurs groupements choisis parmi -OR, -COOR, -COR, -SH, -SR, -OH et les atomes d'halogène ; un résidu polyorganosiloxane modifié ou non ; un groupement polyoxyalkylène; R désignant un groupe hydrocarboné saturé ou non, linéaire, ramifié ou cyclique, comportant de 1 à 20 atomes de carbone et contenant éventuellement un ou plusieurs atomes d'azote, d'oxygène, de soufre, et éventuellement substitué par un ou plusieurs groupements choisis parmi -OR', -COOR', -COR', -SH, -SR', -OH et les atomes d'halogène, ou un résidu de polymère, R' étant un radical alkyle en C₁-C₁₀.

8. Composition selon l'une quelconque des revendications précédentes, dans laquelle R₃ et R₄ sont choisis, indépendamment l'un de l'autre, parmi les groupements -N(R)₃⁺, -S(R)₂⁺, -SH₂⁺, -NH₃⁺, -NO₂, -SO₂R, -C≡N, -COOH, -COOR, -COSR, -CONH₂, -CONHR, -F, -CI, -Br, -I, -OR, -COR, -SH, -SR, -OH, les groupes alcényle linéaires ou ramifiés, les groupes alcynyle linéaires ou ramifiés, les groupements mono- ou polyfluoroalkyle en C₁-C₄, les groupements aryle et aryloxy ; R désignant un groupe hydrocarboné saturé ou non, linéaire, ramifié ou cyclique, comportant de 1 à 20 atomes de carbone et contenant éventuellement un ou plusieurs atomes d'azote, d'oxygène, de soufre, et éventuellement substitué par un ou plusieurs groupements choisis parmi -OR', -COOR', -COR', -SH, -SR', -OH et les atomes d'halogène, ou un résidu de polymère, R' étant un radical alkyle en C₁-C₁₀.

9. Composition selon l'une quelconque des revendications précédentes, dans laquelle le ou les monomères électrophiles sont choisis parmi :
• le benzylidène malononitrile (A), le 2-(4-chloro-benzylidène)-malononitrile (A1), le 2-cyano-3-phényl acrylate d'éthyle (B), le 2-cyano-3-(4-chloro-phényl) acrylate d'éthyle (B1) : 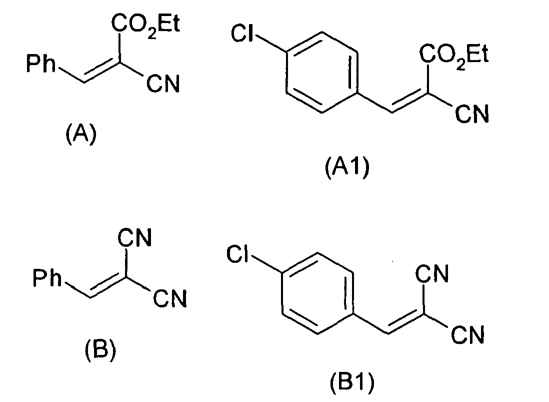
• le 2-méthylène-malonate de diéthyle (C) : 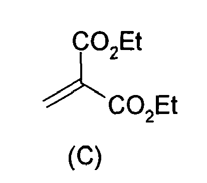
• le 2-éthoxycarbonylméthylèneoxycarbonyl acrylate d'éthyle (D) : 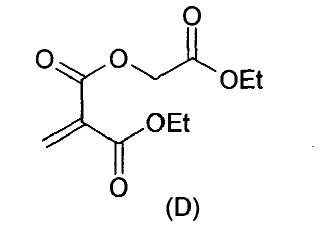
• les dérivés α-(méthylsulfonyl)acrylates de méthyle (K), α-(méthylsulfonyl)acrylates de d'éthyle (L), α-(tert-butylsulfonyl)acrylates de méthyle (M), α-(méthylsulfonyl)acrylates de tert-butyle (N), α-(tert-butylsulfonyl)acrylates de tert-butyle (O), et
• les dérivés 1,1-bis-(méthylsulfonyl)éthylène (P), 1-acétyl-1-méthyl sulfonyl éthylène (Q), α-(méthylsulfonyl) vinyl sulfonate de méthyle (R), α-méthylsulfonylacrylonitrile (S) :

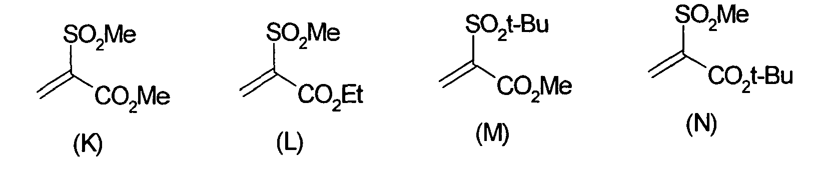

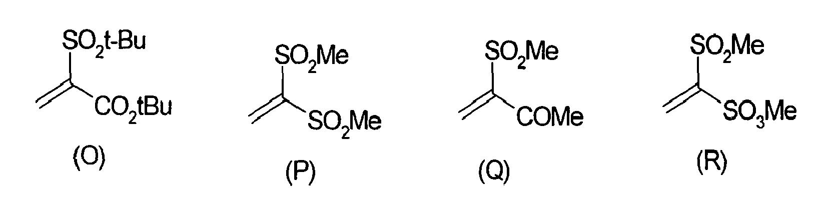

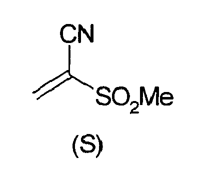

10. Composition selon l'une quelconque des revendications précédentes, dans laquelle le ou les monomères électrophiles sont choisis parmi les monomères de la famille des cyanoacrylates de formule (II) : 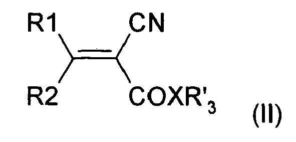 dans laquelle :
- X peut désigner NH, S, O ;
- R'₃ peut désigner un atome d'hydrogène ou un groupe R;
- R, R₁ et R₂ sont tels que définis dans la revendication 7.

11. Composition selon l'une quelconque des revendications précédentes, dans laquelle R₁ et R₂ représentent un atome d'hydrogène.

12. Composition selon la revendication 10 ou 11, dans laquelle X désigne O.

13. Composition selon l'une quelconque des revendications 10 à 12, dans laquelle R'₃ représente un radical alkyle en C₆-C₁₀.

14. Composition selon l'une quelconque des revendications précédentes, dans laquelle le ou les monomères électrophiles sont choisis parmi les monomères de la famille des 2-cyanoacrylates d'alkyle ou d'alcoxyalkyle de formule (V) : 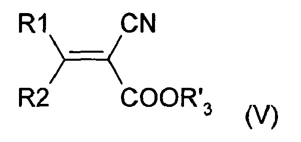 dans laquelle:
- R'₃ représente un radical alkyle en C₁-C₁₀ ou alcoxy(C₁-C₄)alkyle(C₁₋C₁₀);
- R₁ et R₂ sont tels que définis à la revendication 7.

15. Composition selon la revendication 14, dans laquelle R'₃ représente un radical alkyle en C₆-C₁₀.

16. Composition selon la revendication 14 ou 15, dans laquelle R₁ et R₂ représentent un atome d'hydrogène.

17. Composition selon l'une quelconque des revendications précédentes, dans laquelle le ou les monomères électrophiles sont choisis parmi les composés de formule (VI) et leurs mélanges : 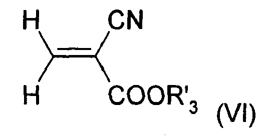 dans laquelle R'₃ est choisi parmi les radicaux suivants:
-(CH₂)₇-CH₃;
-CH(CH₃)-(CH₂)₅-CH₃;
-CH₂-CH(C₂H₅)-(CH₂)₃-CH₃;
-(CH₂)₅-CH(CH₃)-CH₃;
-(CH₂)₄-CH(C₂H₅)-CH₃.

18. Composition selon l'une quelconque des revendications précédentes, dans laquelle le ou les monomères électrophiles sont présents en quantité comprise entre 0,1 % et 80 % en poids environ du poids total de la composition.

19. Composition selon l'une quelconque des revendications précédentes, dans laquelle le milieu cosmétiquement acceptable est anhydre.

20. Composition selon la revendication 1 9, dans laquelle le milieu cosmétiquement acceptable est choisi parmi :
• les alcools aromatiques ;
• les alcools gras ;
• les polyols modifiés ou non modifiés ;
• les silicones volatiles ou non volatiles ;
• les huiles minérales, organiques ou végétales ;
• les cires oxyéthylénées ou non oxyéthylénées, les paraffines, les alcanes ;
• les acides gras, les amides grasses, les esters gras; et leurs mélanges.

21. Composition selon l'une quelconque des revendications précédentes, comprenant de plus au moins un agent nucléophile.

22. Composition selon la revendication 21, dans laquelle l'agent nucléophile est l'eau.

23. Procédé pour la décoloration ou la déformation permanente des fibres kératiniques, comprenant l'application sur les fibres kératiniques une composition oxydante comprenant, dans un milieu cosmétiquement acceptable, au moins un monomère électrophile tel que défini à l'une quelconque des revendications 1 et 7 à 17 et au moins un agent oxydant en présence d'au moins un agent nucléophile.

24. Procédé selon la revendication 23, dans lequel le ou les agents oxydants sont parmi :
• le peroxyde d'hydrogène ou un agent susceptible de produire du peroxyde d'hydrogène par hydrolyse ;
• les persulfates, les perborates, les percarbonates d'ammonium ou de métaux alcalins ;
• les bromates alcalins ;
• le monoperoxyphthalate de magnésium ;
• les nitrites ;
• l'acide périodique et ses sels hydrosolubles ;
• les ferricyanures de métaux alcalins ;
• l'oxyde d'argent ;
• le réactif de Fenton ;
• l'oxyde de plomb (IV);
• les anions d'un métal choisi parmi les permanganates ou les bichromates ;
• les sels métalliques du groupe III à VIII du tableau périodique;
• les sels de terres rares choisis parmi les sels de lanthanides, les sels de cérium Ce³⁺, Ce⁴⁺, les sels de lanthane La³⁺, d'europium Eu²⁺, Eu³⁺, les sels de gadolinium Gd³⁺, les sels d'Ytterbium Yb²⁺, Yb³⁺, de dysprosium Dy³⁺;
• l'hypochlorite de sodium;
• la benzoquinone.

25. Procédé selon la revendication 23 ou 24, dans lequel l'application de la composition oxydante est réalisée en au moins deux étapes, dans lequel une des étapes consiste à appliquer sur les fibres kératiniques une composition comprenant le ou les monomères électrophiles et l'autre étape consiste à appliquer sur les fibres kératiniques une composition comprenant le ou les agents oxydants, l'ordre des étapes étant indifférent.

26. Procédé selon l'une quelconque des revendications 23 à 25, dans lequel l'agent nucléophile est appliqué séparément.

27. Procédé selon l'une quelconque des revendications 23 à 25, dans lequel l'agent nucléophile est présent dans la composition comprenant le ou les agents oxydants.

28. Procédé selon l'une quelconque des revendications 23 à 27, comprenant une étape qui consiste à appliquer sur les fibres kératiniques au moins un agent réducteur de la kératine.

29. Procédé selon la revendication 28, dans lequel le ou les agents réducteurs sont appliqués sur les fibres kératiniques avant la composition comprenant le ou les agents oxydants.

30. Kit pour la décoloration ou la déformation permanente des fibres kératiniques, **caractérisé par le fait qu'**il contient une composition comprenant au moins un monomère électrophile tel que défini à l'une quelconque des revendications 1 et 7 à 17 et au moins un inhibiteur de polymérisation et une composition comprenant au moins un agent oxydant tel que défini à la revendication 23 ou 24.

31. Kit selon la revendication 30, dans lequel la composition comprenant le ou les agents oxydants comprend de plus au moins un agent nucléophile tel que défini à la revendication 21 ou 22.

32. Kit selon la revendication 30, contenant de plus une composition comprenant au moins un agent nucléophile tel que défini à la revendication 21 ou 22.

33. Utilisation pour la décoloration ou la déformation permanente des fibres kératiniques d'une composition oxydante telle que définie à la revendication 23 ou 24.
